# EUROPEAN PATENT APPLICATION

(11) **EP 2 567 689 A1**
(43) Date of publication of application: **13.03.2013**
(21) Application number: 11306137.8
(22) Date of filing: 12.09.2011
(51) Int. Cl.: A61K 9/00, A61K 31/5575, A61K 47/36, A61P 27/06

(54) **Ophthtalmic compositions comprising prostaglandin F2 alpha derivatives and hyaluronic acid**

(71) Applicant: Visiotact Pharma, 75012 Paris (FR)
(72) Inventor: Hadj-Slimane, Réda, 75018 Paris (FR)

(57) **Abstract**

The present invention relates to a preservative-free ophthalmic composition containing at least an analogue of prostaglandin compound and at least a hyaluronic acid for treating ocular hypertension and glaucoma.

## Description

The present invention relates to a preservative-free ophthalmic composition containing at least an analogue of prostaglandin as active compound and at least a hyaluronic acid polymer for treating ocular hypertension and glaucoma.

Glaucoma is an eye disease characterized by damage to the optic nerve head and related visual field defects, often accompanied by elevated intraocular pressure (IOP).

Glaucoma is characterized by the slow, progressive degeneration of retinal ganglion cells and optic nerve axons. The disease affects over 66 million people worldwide, causing bilateral blindness in 6.8 million. One may divide glaucoma in primary glaucoma without known preceding cause, and secondary glaucoma. Primary glaucoma may be genetically determined. The term secondary glaucoma refers to glaucoma caused by some known antecedent or concomitant ocular disease.

Furthermore, primary glaucoma can be classified on anatomic basis into four major divisions: open-angle glaucoma, angle closure glaucoma, mixed glaucoma and congenital glaucoma. Primary open-angle is the most frequent type of glaucoma.

Open-angle glaucoma represents a chronic, slowly progressive optic neuropathy, characterized by progressive excavation of the optic nerve head and a distinctive pattern of visual field defects. The disease is multifactorial in origin and is associated more closely with elevated intraocular pressure (IOP) resulting in the main from reduced drainage of aqueous humor.

There a several recognised risk factors for glaucoma, such as an increased intraocular pressure (IOP), aging, family history, high myopia, systemic hypertension, cardiovascular disease, migraine headaches, peripheral vasospasm and prior nerve damage. The visual damage incurred from glaucoma is considered irreversible. However, it can be treated if diagnosed at an early enough stage.

Medications involve inhibiting the inflow of aqueous humor, enhancing the outflow of aqueous humor, protecting the optic nerves and manipulating the osmotic pressure between plasma and the eyes.

Currently available medications for the treatment of glaucoma belong to several pharmacological classes, including beta-adrenergic blockers (timolol maleate, carteolol, betaxolol), cholinergic agonists, carbonic anhydrase inhibitors (dorzolamide, brinzolamide) or adrenergic receptor blockers (brimonidin).

Another method of reducing IOP is by enhancing the outflow of humor from the eyes through the use of muscarinic acetylcholine receptor agonists. This mechanism is indirect, but involves a muscarinic acetylcholine receptor (M3)-mediated contraction of the ciliary muscle. The contraction causes the widening of the spaces in the trabecular meshwork. The newest class of drugs using this strategy is the prostaglandin F2α derivates (like unoprostone, latanoprost, travoprost or bimatoprost).

All these medications operate under a mechanism whereby the IOP is lowered. These therapies are typically administered as eye drops.

Prostaglandin analogues are chemical moieties, found in tissues or organs of humans, exhibiting a wide range of physiological activities.

Latanoprost, also known as isopropyl-(Z)-7-[(IR,2R,3R,5S)-3,5-dihydroxy-2-[(3R)-3-hydroxy-5-phenylpentyl]cyclopentyl]-5-heptenoate, is a prostaglandin F2α receptor agonist, lowering the IOP by promoting an uveoscleral outflow of aqueous humor (Camras, C. B. et al. Invest. Ophthalmol. Vis. Sci. 28:463-469, 1987). Latanoprost was found to have more excellent effects of lowering intraocular pressure than the β-blockers such as timolol maleate (Aung, T. et al. Ophthalmol. 107:1178-1183, 2000). Such properties of latanoprost make it possible to show a lasting effect of controlling the IOP and a superior effect of lowering the IOP. In addition, the compliance in patients for this drug is high, and its safety has been established by its excellent ocular tolerance (Sudesh, S. et al. Arch. Ophthalmol. 117:539-540, 1999).

Latanoprost, travoprost and bimatoprost have been introduced in the market under the trade marks respectively of Xalatan®, Travatan®, and Lumigan® as ophthalmic eye drop solutions for the treatment of ocular hypertension and glaucoma. Xatlatan® is, to date, the most prescribed anti-glaucoma medicine in the world.

However, problems associated with these prostaglandin analogues are their rather poor water solubility and their chemical instability especially in aqueous solutions. Consequently many different ophthalmic formulations have been proposed to overcome such problems.

Xalatan® contains latanoprost at a concentration of 0.005%. This product is sold in a multidose bottle of 5mL preserved by benzalkonium chloride at 0.02%. Travatan® contains travoprost at a concentration of 0.004%, this product is sold in a bottle of 2.5 or 5mL. Lumigan® contains bimatoprost at a concentration of 0.01% or 0.03%, this product is sold in a bottle of 3mL preserved by benzalkonium chloride at 200 or 500 ppm. Even though this medicine is very appreciated for the treatment of glaucoma, it has side effects, some of which may be due to the presence of the preservative agent. After few months of treatment of this chronic life-long disease, this medicine eye drops may start to irritate and wound the ocular surface.

Preservatives which exhibit sufficient antimicrobial effect on bacteria and fungi have traditionally been used in ophthalmic compositions. In addition to this, the preservatives are required to be stable and preferably to homogenize and stabilize the composition by interacting with the ingredients, by homogeneously dispersing or dissolving the ingredients into the vehicle or base.

Nowadays, the most commonly used preservatives in commercially available ophthalmic solutions are benzalkonium chloride and other quaternary ammonium salts. Benzalkonium chloride is one of most used antimicrobial preservatives for ophthalmic formulations but it has been also widely used in the formation of ophthalmic microemulsions (U.S. Pat. No. 5,698,219) thanks to its positive charge which stabilizes the droplets.

Benzalkonium chloride (alkyl dimethyl benzyl ammonium chloride) is a nitrogen-based quaternary ammonium compound demonstrating broad-spectrum antimicrobial activity. However, there is increasing concern that the use of biocides such as benzalkonium chloride and other quaternary ammonium compounds may be contributing to the development of microorganisms with decreased susceptibilities to antibiotics and disinfectants. Moreover, preservatives are also known as the major etiology of keratoconjunctive disorders.

Indeed, studies have shown the allergenic (Park HJ, Kang HA, Lee JY, Kim HO, "Allergic contact dermatitis from benzalkonium chloride in antifungal year solution," Contact Derm., Vol. 42, No. 5, 2000, p . 306-7) and the toxicity of benzalkonium chloride (Marple B, Roland P, Benninger M, "Safety review of benzalkonium chloride as a preservative used in intranasal solutions: an overview of Conflicting data and opinions," Otolaryngol Head Neck Surg, vol 130, No. 1, 2004, p. 131-41). In addition, a study has shown that repeated use of benzalkonium chloride (at a concentration of 0.02%) distorts the cornea and induces irreversible eye damage (Swan, KC, "Reactivity of the Ocular Tissues to Wetting Agents "Am J Ophthalmol., 27, 118 (1944)).

Finally, a more recent study conducted in 2009 shows that benzalkonium chloride induced resistance of *Pseudomonas aeruginosa* type bacteria to the antibiotic ciprofloxacin (Paul H. Mc Cay, Alain A. Ocampo-Sosa and Gerard Fleming TA, "Effect of subinhibitory concentrations benzalkonium chloride of on the Competitiveness of Pseudomonas aeruginosa grown in continuous culture, "Microbiology 156 (2010), 30-38).

Since latanoprost is practically insoluble in water, it is necessary to solve also the problem of solubility to water in order to formulate the latanoprost in ophthalmic solutions, especially for unit dose preparations. In EP 1 321 144 and US 2007/248697, a non-ionic surfactant has been added to the ophthalmic solution. Other attempts to compensate the difficulties in formulating highly lipophilic prostaglandin analogues in water have been described for example in EP 0 969 846, EP 1 666 043, EP 1 011 728 and WO 2007/042262 but they do not mention any preservative-free composition.

There is still a need in ophthalmologic prostaglandin analogues products which are at least as efficient as the commercial products, which present an enhanced chemical stability of prostaglandin analogues, which are less toxic, more physically and chemically stable than conventional products, i.e. which are stable overtime and which present a good tolerability for the patient. By good tolerability, it is understood in the present invention, that the ratio of therapeutic benefit to ocular discomfort is acceptable by the patient.

It is therefore an object of the present invention to provide an ophthalmic aqueous solution comprising prostaglandin analogues compounds and free of preservatives, wherein said prostaglandin analogue remains soluble, stable and bioavailable in the preservative-free preparation. The ophthalmic solution according to the invention provides a significant clinical advantage as there is currently an unmet clinical need of preservative-free prostaglandin eye drops for ocular hypertension and/or glaucoma patients.

The applicant formulated a new ophthalmic composition for use in treating ocular hypertension and/or glaucoma, wherein the ophthalmic composition contains at least a prostaglandin analogue compound and at least hyaluronic acid having a molecular weight comprised between 1,600,000 and 4,000,000 Da, said composition being free of preservative.

By ophthalmic, it is meant a composition intended to be administered to the eye and which presents a pharmaceutical effect. Preferably, the composition is topically applied.

The term "free of" in combination with a compound means that the ophthalmic composition does not contain absolutely any compounds of that kind, or the composition contains preservatives at a concentration that is undetectable or does not provide a preservative effect.

By prostaglandin analogue compound, is mean prostaglandin F2α analogs such as 17-phenyl-13,14 dihydro trinor prostaglandin F2α isopropyl ester (latanoprost), 20-ethyl prostaglandin F2α, (+)-fluprostenol isopropyl ester (travoprost), 17-phenyl trinor prostaglandin F2α amide, 17-phenyl-13,14 dihydro trinor prostaglandin F2α ethyl amide (bimatoprost), tafluprost prostaglandin F 2α ethanolamide, bimatoprost (free acid)-d 4, bimatoprost-d 4 , latanoprost ethyl amide, 13,14 dihydro-15-keto-20-ethyl prostaglandin F2α (unoprostone), 13,14 dihydro-15-keto-20-ethyl prostaglandin F2α isopropyl ester (unoprostone isopropyl ester), 8-isoprostaglandinE2, or a mixture of two or more thereof.

Preferably, the prostaglandin analogue compound is latanoprost.

Latanoprost, travoprost, bimatoprost, tafluprost, unoprostone isopropyl, 8-isoprostaglandinE2, like most of the prostaglandin analogues, are almost insoluble in water.

In the present application, percentages are expressed in weight relative to the total volume of the composition (% w/v). In the composition of the invention, the content of the prostaglandin analogue compound is between 0.001 and 0.1% (w/v). The content of the hyaluronic acid is between 0.01 and 1.0% (w/v), preferably between 0.1 and 0.2% (w/v), and even more preferably between 0.10 % and 0.15 % (w/v).

The hyaluronic acid molecular weight in the composition of the invention ranges from 1.6 to 4.0 MDa. Preferably, the hyaluronic acid molecular weight ranges from 1.6 to 2.4 MDa.

In the compositions of the invention, the chemical stability of latanoprost, travoprost and bimatoprost in particular, is enhanced.

The prostaglandin analogue may be administered topically in a form of eye drops, eye ointment, ophthalmic gel or eye solution for injection. Preferably, the composition containing the prostaglandin compound is sterile, and may be in a single dose or a multidose form.

Preferably, the ophthalmic composition of the invention is administered at least once a day.

Combinations of prostaglandins analogue compounds and other eye drugs are also possible, in particular with other classes of anti-glaucoma agents. They could, for example, be beta-blockers like timolol maleate or carteolol chloride, carbonic anhydrase inhibitors such as dorzolamide chloride, or alpha-adrenergic agonists such as brimonidine tartrate. Among them, the combination of prostaglandin analogues compounds with timolol maleate is preferred.

In practice, these anti-glaucoma agents may form between 0.1 and 0.5% by weight of the solution.

The ophthalmic composition according to the invention may also comprise conventional adjuvants or excipients used in ophthalmic compositions, such as buffering agents, solvents, pH adjusters, tonicity agents and the like.

Examples of suitable buffering agents include but are not limited to sodium dihydrogen phosphate dihydrate, boric acid or a salt thereof (borax, etc), citric acid or a salt thereof (like sodium citrate, etc), tartaric acid or a salt thereof (sodium tartrate, etc), gluconic acid or a salt thereof (like sodium gluconate), acetic acid or a salt thereof (sodium acetate, etc), phosphoric or a salt thereof (sodium monohydrogen phosphate, etc), various amino acids such as glutamic acid and ε-aminocaproic acid and tris buffers, and a combination thereof.

Specific examples of tonicity agents include but are not limited to glycerol, sorbitol, mannitol, propylene glycol, glycerin, erythriol, arabitol, xylitol, ribitol, galactitol, multitol, lactitol and other sugar alcohols, sodium chloride, potassium chloride and calcium chloride. Among them, sorbitol or mannitol is preferred.

The pH of the ophthalmic aqueous solution according to the invention is preferably from 4 to 10. In consideration of irritation to the eyes, the aqueous pharmaceutical composition of the invention may be preferably adjusted to pH 5.0 to 8.0, and more preferably 6.2 to 7.2.

Any pH adjustor can be used, like phosphates or borates. More specifically, sodium hydroxide, potassium hydroxide, sodium carbonate, sodium hydrogen carbonate, sodium bicarbonate, boric acid or a salt thereof (borax), hydrochloric acid, citric acid or a salt thereof (sodium citrate, sodium dihydrogen citrate etc.), phosphoric acid or a salt thereof (disodium hydrogen phosphate, potassium dihydrogen phosphate etc.), acetic acid or a salt thereof (sodium acetate, ammonium acetate etc.), and tartaric acid and/or hydrochloric acid may be used or a salt thereof.

To adjust the osmotic pressure ratio of the aqueous pharmaceutical composition according to the invention, a variety of isotonic agents generally used may be added.

Examples of the ionic isotonic agent include potassium chloride, calcium chloride, sodium chloride, magnesium chloride and the like. Examples of the nonionic isotonic agent include dextrose, xylitol, D-sorbitol, D-mannitol, glycerol, propylene glycol, macrogol 4000 and the like.

Other ingredients such as a decongestant, an eye muscle accommodator, an antiphlogistic/styptic, a vitamin, amino acid and the like may be added, provided that the ingredient does not cause a problem such as eye irritation.

Examples of decongestant include epinephrine, epinephrine hydrochloride, ephedrine hydrochloride, tetrahydrozoline hydrochloride, naphazoline hydrochloride, naphazoline nitrate, phenylephrine hydrochloride, methyl ephedrine hydrochloride, and the like.

Examples of eye muscle accommodator include neostigmine methylsulfate and the like.

Examples of antiphlogistic/styptic include epsilon-aminocaproic acid, allantoin, berberine chloride, berberine sulfate, sodium azulene sulfonate, dipotassium glycyrrhizinate, zinc sulfate, zinc lactate, lysozyme chloride, and the like.

Examples of vitamin include flavin adenine dinucleotide sodium, cyanocobalamin, retinol acetate, retinol palmitate, pyridoxine hydrochloride, panthenol, calcium pantothenate, sodium pantothenate, tocopherol acetate, and the like.

Examples of amino acid include potassium L-asparaginate, magnesium L-asparaginate, magnesium/potassium L-asparaginate (equal parts mixture), aminoethyl sulfonic acid, sodium chondroitin sulfate, and the like.

The ophthalmological composition of the present invention can be systemically administered in an injectable dosage form or locally administered as an eye drop.

Since degradation of prostaglandin F2a analogues is remarkably reduced in the compositions of the invention, these ophthalmological compositions are useful, especially in the case of eye drops presented in multidosis container which may be used for a certain period of time.

The ophthalmic compositions of the invention may be conditioned in monodosis containers or in multidosis containers. Unit dose containers containing the ophthalmic composition according to the invention are preferred.

Generally, a unit dose ophthalmic container manufactured by blow moulding method has a volume of about 1 ml and about 0.2 to 0.5 ml of solution is filled. A large variety of shapes are known in such containers. Typical examples are seen in US 5,409,125 and US 6,241,124. The container may have a cylindrical shape. It is possible to appropriately select a capacity of the container in the case of a multi-dose container, for example, from a range of about 0.5 to 20 mL, and the capacity may preferably be 1 to 10 mL, more preferably 2 to 5 mL. Further, the eye drop preparation container may be covered with a shrinkable film as required after packing the eye drop preparation composition of this invention.

It is possible to use general plastic containers for eye drop preparations for packing the eye drop preparation composition. For example, as a material for the container, it is possible to use a thermoplastic resin such as polyethylene, polypropylene, polyethylene terephthalate, polyethylene naphthalate, polyallylate, and a polycarbonate ethylene/vinyl alcohol copolymer insofar as the thermoplastic resin is satisfactory in terms of cost, strength, optical transmittance, gas or water vapor barrier property (moisture permeation), and the like. Examples of a preferred polymer alloy include a polymer blend of a plurality of synthetic resins (polymer blend of polyethylene terephthalate and polyethylene naphthalate, etc.).

It is possible to select dosage and administration of the ophthalmic solution of this invention depending on symptom of a glaucoma patient requiring instillation, the active ingredients contained in the ophthalmic solution, and formulation. A typical administration of the composition of the invention is by instillation of about one drop once per day.

The material of the filter on the top of the container may be polyether sulfone (PES), polyvinylidene fluoride (PVDF), polycarbonate, polytetrafluoroethylene (PTFE) or mixed cellulose ester (MSE). In particular, polyether sulfone is more preferable. The commercially available filters made from polyether sulfone include Millipore Express^{®} and Millipore Express PLUS^{®} made by Millipore Corporation.

The preservative-free ophthalmic composition according to the invention can be stored at room temperature, including unit dose pipettes and dispensers. Stability studies have shown that a preservative-free ophthalmic latanoprost composition according to the invention is stable for a long time, at least for one year, preferably two years and more preferably three years. The same result can be observed for compositions containing travoprost or bimatoprost.

The invention is further illustrated by the examples below.

### Example 1: Chemical Stability Studies:

In order to understand the intrinsic stability mechanism of the molecule by establishing degradation pathways, the Applicant has developed stress stability testing during which the compositions of the invention are subjected to a temperature of 25 to 40°C during specified period of time.

Briefly, Latanoprost (0.05 mg/mL), Travoprost (0.04 mg/mL), Bimatoprost (0.3 mg/mL) were respectively mixed with hyaluronic acid possessing different molecular weight at 0.15% in 100 mL of a phosphate buffer solution containing 0.317 g of Na₂HPO₄, 12H₂0 0.067 g of Na₂HPO₄, 2H₂0, 4.5 g of D-sorbitol and 0.05 g of EDTA in water.

A range of hyaluronic acid (1.6 to 2.4 Mda) is used to establish stability. All prostaglandin samples are prepared using dilution of Latanoprost (0.005 g), Travoprost (0.004 g), Bimatoprost (0.03 g) in safe condition to obtain clear and colourless solution free of particles using phosphate buffer with a control osmolarity (240-290 mosmol/kg) and equilibrated PH (6.3 to 7.1). Latanoprost, Travoprost and Bimatoprost are purchased from ACETO FRANCE SAS and Teva API Division. Hyaluronic acid is purchassed from Shiseido.

The stability study was performed on samples at different temperatures: in refrigeration (2°C - 8°C), at 25°C, at 30°C and at 40°C. Assessment of Latanoprost, Travoprost, Bimatoprost were performed by means of High Pressure Liquid Chromatography (HPLC). Briefly, samples were preserved in a glass container. Latanoprost, Travoprost and Bimatoprost contents were determined with an HPLC (Shimadzu) equipped with an UV detector and using a mixture of two mobile phases and using shimadzu class-VP software. The data reported in the tables are expressed as Latanoprost, Travoprost and Bimatoprost percentage recovery.

**Table 1: Latanoprost recovery during time and temperature variations**

| Latanoprost without polymer (0.05 mg/mL phosphate buffer) | | | | |
|---|---|---|---|---|
| Time (Month) | 2-4°C | 25°C | 30°C | 40°C |
| 0 | 88.7+/-0.2 | 88.7+/-0.2 | 88.7+/-0.1 | 88.7+/-0.3 |
| 1 | 88.1+/-0.4 | 88.2+/-1.1 | 87.1+/-0.7 | 81.0+/-0.5 |
| 2 | 88.2+/-0.3 | 85.9+/-1.9 | 85.3+/-1.6 | 75.6+/-0.1 |
| 3 | 88.4+/-0.7 | 56.8+/-0.9 | 55.4+/-1.3 | 48.5+/-3.2 |

The latanoprost alone is not stable since after 3 months at 30 or 40°C, almost half of the compound is degraded.

**Table 2: Latanoprost recovery in the presence of HA (1.6 MDa) at 0.15% (w/v)**

| Latanoprost/HA-1.6 MDa (0.05mg/mL phosphate buffer) | | | | |
|---|---|---|---|---|
| Time (Month) | 2-4°C | 25°C | 30°C | 40°C |
| 0 | 99.1+/-0.5 | 99.1+/-0.4 | 99.1+/-0.1 | 99.1+/-0.1 |
| 1 | 99.0+/-0.9 | 98.9+/-0.7 | 98.6+/-0.8 | 98.7+/-1.1 |
| 2 | 98.8+/-0.4 | 98.9+/-0.7 | 98.9+/-0.4 | 98.3+/-1.4 |
| 3 | 98.0+/-0.9 | 96.9+/-0.4 | 97.1+/-0.2 | 96.0+/-0.1 |

**Table 3: Latanoprost recovery in the presence of HA (2.4 MDa) at 0.15% (w/v)**

| Latanoprost/HA-2.4 MDa (0.05 mg/mL phosphate buffer) | | | | |
|---|---|---|---|---|
| Time (Month) | 2-4°C | 25°C | 30°C | 40°C |
| 0 | 99.3+/-0.7 | 99.3+/-0.6 | 99.3+/-0.5 | 99.3+/-0.2 |
| 1 | 98.7+/-0.5 | 98.4+/-0.2 | 98.6+/-0.7 | 98.1+/-1.4 |
| 2 | 98.6+/-0.3 | 98.6+/-0.7 | 98.7+/-0.3 | 97.6+/-0.2 |
| 3 | 98.5+/-0.9 | 98.0+/-1.4 | 98.1+/-1.6 | 96.3+/-0.5 |

On the contrary, the recovery of Latanoprost after storage at 25°C at 3 months is at least 97% in presence of HA at molecular weight of 1.6 or 2.4 MDa.

**Table 4: Travoprost recovery in the presence of HA (1.6 MDa) at 0.15% (w/v)**

| Travoprost (0.04 mg/mL phosphate buffer) | | | | |
|---|---|---|---|---|
| | Without polymer | | HA (1.6MDa) | |
| Time (month) | 25°C | 40°C | 25°C | 40°C |
| 0 | 100+/-0.4 | 100+/-0.9 | 100+/-0.1 | 100+/-0.2 |
| 1 | 98.3+/-1.4 | 81.5+/-0.2 | 99.0+/-0.4 | 99.1+/-0.1 |
| 2 | 92.0+/-1.0 | 78.9+/-0.8 | 99.9+/-0.1 | 98.6+/-0.8 |
| 3 | 66.4+/-0.9 | 42.1+/-1.6 | 99.2+/-0.6 | 98.2+/-0.6 |

**Table 5: Bimatoprost recovery in the presence of HA (1.6 MDa) at 0.15% (w/v)**

| Bimatoprost (0.3 mg/mL phosphate buffer) | | | | |
|---|---|---|---|---|
| | Without polymer | | HA (1.6MDa) | |
| Time (weeks) | 25°C | 40°C | 25°C | 40°C |
| 0 | 100+/-1.0 | 100+/-0.4 | 100+/-0.5 | 100+/-0.6 |
| 3 | 94.0+/-0.8 | 92.0+/-0.4 | 99.4+/-0.1 | 98.2+/-0.5 |
| 6 | 93.2+/-0.2 | 91.2+/-0.8 | 98.2+/-0.8 | 97.2+/-0.2 |
| 9 | 88.8+/-0.1 | 85.2+/-0.5 | 99.6+/-0.4 | 97.8+/-0.3 |

The same results are observed for travoprost and bimatoprost (recovery of at least 97-98 % at 40°C).

### Example 2: recovery of prostaglandin analogues in the presence of HA at different molecular weights.

Latanoprost (0.05 mg/mL), Travoprost (0.04 mg/mL), Bimatoprost (0.3 mg/mL) were respectively mixed with hyaluronic acid at 0.15% in solution containing Na₂HPO₄, 12H₂0 (0.317 g), Na₂HPO₄, 2H₂0 (0.067 g), D-sorbitol (4.5 g), EDTA (0.05 g) and purified water qs 100 mL.

A range of hyaluronic acid (1.0 to 2.4 MDa) is used to establish prostaglandin stabilities. Assessment of Latanoprost, Travoprost, Bimatoprost were performed by means of Chromatography of Liquids (HPLC). Briefly, samples were preserved in a glass container. Latanoprost, Travoprost and Bimatoprost contents were determined with an HPLC (Shimadzu) equipped with an UV detector and using a mixture of two mobile phases and using shimadzu class-VP software.

**Table 6 : Prostaglandin analogues recovery (%) in phosphate buffer containing hyaluronic acid (HA) at 0.15%. (w/v)**

| Active substance | HA (1.0 Mda) | HA (1.2 Mda) | HA (1.5 Mda) |
|---|---|---|---|
| Latanoprost | 86+/-0.2 | 87+/-0.1 | 88+/-0.5 |
| Travoprost | 83.5+/-0.4 | 86+/-0.7 | 90.1+/-0.1 |
| Bimatoprost | 87+/-0.1 | 87.2+/-0.3 | 87.4+/-0.2 |
| Active substance | HA (1.6 Mda) | HA (2.0 Mda) | HA (2.4 Mda) |
| Latanoprost | 97+/-0.1** | 98+/-0.4 | 98.5+/-0.5 |
| Travoprost | 98.5+/-0.1** | 98.6+/-0.3 | 98.5+/-0.5 |
| Bimatoprost | 97.1+/-0.3** | 97.5+/-0.2 | 98+/-0.4 |

This Table shows that thee recovery of prostaglandin is very increased when the molecular weight of HA in the solution is above 1.5 MDa.
** means that difference between 1.5 and 1.6 Mda of hyaluronic acid is statistically significant to increase prostaglandin analogue stability (P<0.01)

### Example 3: 0.005% Latanoprost/0.15% Hyaluronic acid ophthalmologic solution

| Substance | Function | Composition per 100 mL |
|---|---|---|
| Latanoprost | Active substance | 0.005 g |
| Hyaluronic acid 1.6 MDa | Solubilizer and stabilizer | 0.15 g |
| Sodium dihydrogen phosphate monohydrate | Buffer | 0.460 g |
| Disodium phosphate anhydrous | Buffer | 0.474 g |
| Sodium chloride | Hypertonicity agent | 0.410 g |
| Purified water | vehicle | Qs 100 mL |

### Example 4: 0.004% Travoprost/0.15% Hyaluronic acid ophthalmologic solution

| Substance | Function | Composition per 100 mL |
|---|---|---|
| Travoprost | Active substance | 0.004 g |
| Hyaluronic acid 1.6 MDa | Solubilizer and stabilizer | 0.15 g |
| Sodium dihydrogen phosphate monohydrate | Buffer | 0.460 g |
| Disodium phosphate anhydrous | Buffer | 0.474 g |
| Sodium chloride | Hypertonicity agent | 0.410 g |
| Purified water | vehicle | Qs 100 mL |

### Example 5: 0.03% Bimatoprost/0.15% Hyaluronic acid ophthalmologic solution

| Substance | Function | Composition per 100 mL |
|---|---|---|
| Bimatoprost | Active substance | 0.03 g |
| Hyaluronic acid 1.6 MDa | Solubilizer and stabilizer | 0.15 g |
| Sodium dihydrogen phosphate monohydrate | Buffer | 0.460 g |
| Disodium phosphate anhydrous | Buffer | 0.474 g |
| Sodium chloride | Hypertonicity agent | 0.410 g |
| Purified water | vehicle | Qs 100 mL |

### Example 6: 0.005% Latanoprost/0.5% Timolol/0.15% Hyaluronic acid ophthalmologic solution

| Substance | Function | Composition per 100 mL |
|---|---|---|
| Latanoprost | Active substance | 0.005 g |
| Timolol maleate | Active substance | 0.68 g |
| Hyaluronic acid 1.6 MDa | Solubilizer and stabilizer | 0.15 g |
| Sodium dihydrogen phosphate monohydrate | Buffer | 0.460 g |
| Disodium phosphate anhydrous | Buffer | 0.474 g |
| Sodium chloride | Hypertonicity agent | 0.410 g |
| Purified water | vehicle | Qs 100 mL |

### Example 7: 0.004% Travoprost/0.5% Timolol/0.15% Hyaluronic acid ophthalmologic solution

| Substance | Function | Composition per 100 mL |
|---|---|---|
| Travoprost | Active substance | 0.004 g |
| Timolol maleate | Active substance | 0.68 g |
| Hyaluronic acid 1.6 MDa | Solubilizer and stabilizer | 0.15 g |
| Sodium dihydrogen phosphate monohydrate | Buffer | 0.460 g |
| Disodium phosphate anhydrous | Buffer | 0.474 g |
| Sodium chloride | Hypertonicity agent | 0.410 g |
| Purified water | vehicle | Qs 100 mL |

### Example 8: 0.03% Bimatoprost/0.5% Timolol/0.15% Hyaluronic acid ophthalmologic solution

| Substance | Function | Composition per 100 mL |
|---|---|---|
| Bimatoprost | Active substance | 0.03 g |
| Timolol maleate | Active substance | 0.68 g |
| Hyaluronic acid 1.6 MDa | Solubilizer and stabilizer | 0.15 g |
| Sodium dihydrogen phosphate monohydrate | Buffer | 0.460 g |
| Disodium phosphate anhydrous | Buffer | 0.474 g |
| Sodium chloride | Hypertonicity agent | 0.410 g |
| Purified water | vehicle | Qs 100 mL |

### Example 9: 0.005% Latanoprost /0.10% Hyaluronic acid Ophthalmologic solution

| Substance | Function | Composition per 100 mL |
|---|---|---|
| Latanoprost | Active substance | 0.005 g |
| Hyaluronic acid 1.6 MDa | Solubilizer and stabilizer | 0.10 g |
| Sodium dihydrogen phosphate monohydrate | Buffer | 0.460 g |
| Disodium phosphate anhydrous | Buffer | 0.474 g |
| Sodium chloride | Hypertonicity agent | 0.410 g |
| Purified water | vehicle | Qs 100 mL |

## Claims

1. An ophtalmic composition comprising at least an analogue of prostaglandin compound and at least a hyaluronic acid having a molecular weight comprised between 1,600,000 and 4,000,000 Da, said composition being free of preservative.

2. The ophtalmic composition according to claim 1, in which the hyaluronic acid has a molecular weight comprised between 1,600,000 and 2,400,000 Da.

3. The ophtalmic composition according to anyone of claims 1 to 2, wherein the prostaglandin analogue compound is selected from the group consisting of 17-phenyl-13,14 dihydro trinor prostaglandin F2α isopropyl ester (latanoprost), 20-ethyl prostaglandin F2α, (+)-fluprostenol isopropyl ester (travoprost), 17-phenyl trinor prostaglandin F2α amide, 17-phenyl-13,14 dihydro trinor prostaglandin F2α ethyl amide (bimatoprost), tafluprost prostaglandin F2α ethanolamide, bimatoprost (free acid)-d 4, bimatoprost-d4, latanoprost ethyl amide, 13,14 dihydro-15-keto-20-ethyl prostaglandin F2α (unoprostone), 13,14 dihydro-15-keto-20-ethyl prostaglandin F2α isopropyl ester (unoprostone isopropyl ester), 8-isoprostaglandinE2, or a mixture of two or more thereof.

4. The ophtalmic composition according to claim 3, wherein the prostaglandin analogue compound is latanoprost.

5. The composition according to anyone of claims 1 to 4, wherein the content of the prostaglandin analogue compound is between 0.001 and 0.1% (w/v) in weight based on the total volume of the ophtalmic composition.

6. The ophtalmic composition according to anyone of claims 1 to 5, wherein the content of the hyaluronic acid is between 0.01 and 1.0% (w/v) based on the total ophtalmic composition.

7. The ophtalmic composition according to anyone of claims 1 to 6, wherein the content of the hyaluronic acid is between 0.1 and 0.20% (w/v) based on the total ophtalmic composition.

8. The ophtalmic composition according to anyone of claims 1 to 7, wherein the content of the hyaluronic acid is between 0.1 % and 0.15 % (w/v) based on the total ophtalmic composition.

9. The ophtalmic composition according to anyone of claims 1 to 8, wherein the ophtalmic composition is in a form of eye drops, eye ointment, ophthalmic gel or eye formulation for injection.

10. The ophtalmic composition according to anyone of claims 1 to 9, which is sterile.

11. The ophthalmic composition according to any one of claims 1 to 10, in a single dose form or a multidose form.

12. The ophtalmic composition according to anyone of claims 1 to 11, wherein it further comprises at least one adjuvant among those selected from the group consisting of buffering agents, solvents, pH adjusters, tonicity agents.

13. The ophthalmic composition according to anyone of claims 1 to 12, further comprising other anti-glaucoma agents, and preferentially Timolol maleate.

14. The ophtalmic composition according to anyone of claims 1 to 13, having a pH from 4 to 10.

15. The ophtalmic composition according to anyone of claims 1 to 14, further containing at least one compound among decongestant, eye muscle accommodator, antiphlogistic/styptic, vitamin and amino acid.

16. The ophtalmic composition according to any one of claims 1 to 15, for its use against ocular hypertension and/or glaucoma.

17. The ophtalmic composition according to claim 16, comprising administering said ophtalmic composition at least once a day.
